# EUROPEAN PATENT APPLICATION

(11) **EP 0 801 083 A2**
(43) Date of publication of application: **15.10.1997**
(21) Application number: 97302276.7
(22) Date of filing: 03.04.1997
(51) Int. Cl.: C08F 8/30, C07K 1/04

(54) **Improved functionalized resin for chemical synthesis**

(30) Priority: 08.04.1996 US 15206
(71) Applicant: PFIZER INC., New York, N.Y. 10017 (US)
(72) Inventor: Estep, Kimberly Gail, Stonington, Connecticut 06378 (US); Roskamp, Eric J., Old Lyme, Connecticut 06371 (US)
(74) Representative: Moore, James William

(57) **Abstract**

A support functionalized by an indole or pyrrole aldehyde is useful for the solid phase synthesis of amide type compounds.

## Description

### Background of the Invention

This invention relates to an improved functionalized support for synthesis of chemical compounds which is particularly useful for preparation of small samples for biological screening. The improved supports are especially useful for rapid synthesis in combination with automated evaluation of pharmaceutical compounds.

The methodology of discovery and development of pharmaceutical chemicals has been extensively reviewed. See, for example, Burger's Medicinal Chemistry, Manfred E. Wolff, Editor, John Wiley & Sons (1980) and Comprehensive Medicinal Chemistry, Corwin Hansch, Editor, Pergamon Press (1990). Random screening has proved to be the most useful method and virtually all presently known pharmaceuticals have been derived from leads discovered by screening of synthetic or natural products.

Cost of random screening is divided between the synthesis of test compounds and biological screening. In recent years, in vitro biological screens have been developed which are relatively quick and inexpensive to carry out. Cell membrane receptors isolated from organs such as the brain, heart, blood vessels, lungs and intestines permit researchers to screen 600-1000 compounds per day with a battery of more than 50 receptors.

M. J. Cain, et al., describes an "Endothelial-1 Receptor Binder, assay for High Throughput Chemical Screening". J. Cardiovas Pharmacol. Vol 17 (Supp 7) 1991. This assay is designed to screen synthetic compounds, natural products and microbiological broths for potential pharmacological activity at the endothelial receptor. The assay is suitable for automation because it is stable and reproducible.

On the other hand, the preparation of isolated, analytically pure samples of new chemical compounds for biological screening has not declined in cost or in time expended. Cain, op. cit., page 335 estimated the cost of custom preparation of a new chemical structure as $3000 in 1980. Present day costs are estimated to range from $5000 to $6000.

Recent advances in robotics, miniaturization and automation have resulted in the development of rapid, high throughput biological screening assays, which can quickly exhaust available sources of chemical diversity. Driven by these advances in biological testing, several methods of generating chemical diversity, primarily peptide- or nucleotide-based oligomer libraries, have been developed using multiple, simultaneous chemical synthesis (Dower, W. J. and Fodor, S. P. A. (1991) Annu. Rep. Med. Chem. **26**, 271-280; Fodor, S. P. A., Read, J. L., Pirrung, M. C., Stryer, L., Lu, A. T. and Solas, D. (1991) Science **251**, 767-773; Jung, G. and Beck-Sickinger, A. G. (1992) Agnew. Chem. Int. Ed. Engl. **31**, 367-383; and Zuckermann, R. N., Kerr, J. M., Siani, M. A. , Banville, S. C. and Santi, D. V. (1992) Proc. Natl. Acad. Sci. USA **89**, 4505-4509).

Houghten, in United States Patent 4,631,211 describes an apparatus for conducting sequential, solid phase synthesis which comprises a foraminous container that encloses reactive particles which are larger than the foraminae and have a known amount of covalently linked organic compound which is capable of reacting during organic synthesis.

DeWitt, et al. (Proc. Natl. Acad. Sci., USA **90**, 6909-13 (1993)) have developed an apparatus and method for the multiple, simultaneous synthesis of organic compounds to create unique collections of compounds. The apparatus and method increases the flexibility and diversity of structures that can be produced by multiple, simultaneous synthesis technology. The target compounds are simultaneously, but separately, synthesized on a solid support in an array format to generate an ensemble of structurally related compounds. See U.S. Patent 5,324,483.

Solid phase synthesis was first described by Merrifield (Merrifield, R. B. (1963) J. Am. Chem. Soc. **85**, 2149-2154) in 1963. Typically, polystyrene beads, 30-70 µm in size are functionalized, e.g. chloromethylated, aminomethylated, or treated in such a way that reagents or organic substrates can be attached to the polymer. Any type of chemistry, compatible with the existing functionality, can be carried out on the organic substrate that is attached to the solid phase. The major advantage of this type of solid phase synthesis is that excess reagents can be used to drive reactions to completion, and that any excess or residual solvent, reagent, or side product that is in solution is simply removed from the polymer by filtering and washing the polymer which is insoluble. Solid phase synthesis has been carried out on a wide variety of solid phases, e.g. , Merrifield resin, Wang resin, Rink resin, TentaGel®, and others.

### Summary of the Invention

This invention relates to a support functionalized with an indole aldehyde which is useful in automated, sequential synthesis of organic amide-like compounds. The support is particularly useful for preparing small samples for biological screening.

This invention provides a compound of the formula 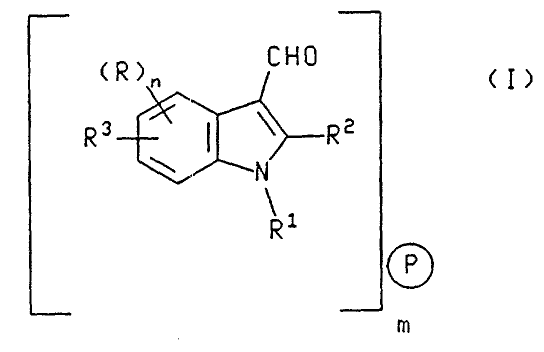 wherein
Ⓟ is a support;
one of R¹, R² and R³ is a linking means adapted to linking to Ⓟ ;
two of R¹, R² and R³ which are not a linking means are independently selected from H, C₁-C₆ alkyl and C₁-C₆ alkoxy;
each R is independently selected from F, Cl, Br, C₁-C₆ alkyl and C₁-C₆ alkoxy;
n is zero to three; and
m is a number such that 0.1 to 1.0 milliequivalent aldehyde is present for each gram of support.

This invention also provides a compound of the formula: 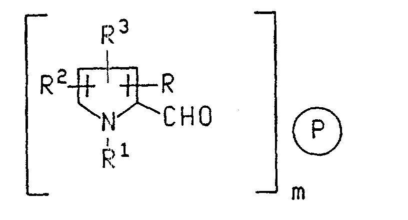 wherein:
Ⓟ is a support;
one of R¹, R² and R³ is a linking means adapted to linking to Ⓟ ;
two of R¹, R² and R³ which are not a linking means are independently selected from H, C₁-C₆ alkyl and C₁-C₆ alkoxy;
each R is independently selected from H, F, Cl, Br, C₁-C₆ alkyl and C₁-C₆ alkoxy;
m is a number such that 0.1 to 1.0 milliequivalent aldehyde is present for each gram of Ⓟ .
In a preferred aspect, this invention provides a compound of the formula II 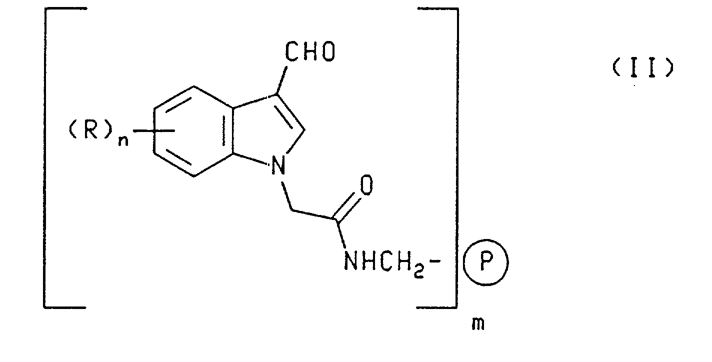
wherein
Ⓟ is a support;
each R is independently selected from F, Cl, Br, C₁-C₆ alkyl and C₁-C₆ alkoxy;
n is zero to four; and
m is a number such that 0.1 to 1.0 milliequivalent aldehyde is present for each gram of said support.

In another aspect this invention provides a compound of Formula I wherein n is zero.

In yet another aspect this invention provides a compound of Formula I wherein n is zero and approximately 0.75 milliequivalents of aldehyde are present for each gram of resin.

In yet another aspect this invention provides a compound of Formula I wherein said support is polystyrene, Wang Resin or Rink resin. Suitable resins are commercially available, for example from Calbiochem-Novabiochem Corp., San Diego, CA.

In another aspect this invention provides a method for the synthesis of an amide type compound which comprises:
a) reacting a primary amino compound under reducing conditions with the compound of Formula I;
b) reacting the product of step (a) with an acid, acid chloride, acid anhydride, sulfuryl chloride, thiourea, chloroformate, or isocyanate;
c) cleaving the product of step (b) to yield said amide type compound.

In another aspect, this invention provides a compound of the formula 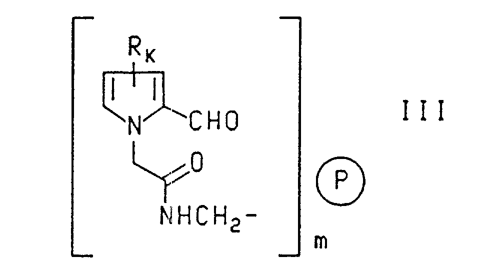 wherein Ⓟ is a support;
K is 0, 1 or 2;
each R is independently selected from F, Cl, Br, C₁-C₆ alkyl and C₁-C₆ alkoxy; and
m is a number such that 0.1 to 1.0 milliequivalents aldehyde is present for each gram of Ⓟ .

In another aspect of this invention provides a method for the synthesis of an amide type compound which comprises
a) reaching a primary amino compound under reducing conditions with a compound of formula III;
b) reaching the product of step (a) with an acid chloride;
c) cleaving the product of step (b) to yield said amide type compound.

### Detailed Description of the Invention

The indole aldehydes of this invention are useful for sequential, automated synthesis of potentially biologically active compounds for screening. As illustrated in Example 4 the compounds of the invention are useful for preparing N-acylated amphetamines which have shown biological activity such as increasing the release of 5-hydroxytryptamine. See Fristrom, et al. Acta Pharmacol. Toxicol (1977), 41(3), 218-24.

As used in this application "a linking means" means an atom or combination of atoms which is capable of forming covalent bonds between a support and an indole or pyrrole aldehyde. Such linking means include any small divalent organic radical containing functional groups such as esters, ethers, amides, sulfonamides, thioesters, thioethers, thioamides and polyglycols. Preferred linking means include -(CH₂)ₗCONH-; -(CH₂)ₗCOO- and -(CH₂)ₘCONH(CH₂)ₗ- wherein I and m are 0 to 4; and -O-CH₂C₈H₄- OCH₂C₆H₄-O-CH₂- and -NH-CH(2,4-di-CH₃O-C₆H₃)-C₆H₄-OCH₂-.

Linking means are described by Fruchlel and Jung Agnew. Chem. Int. Ed. Engl. 1996 **35**, 1742 which is incorporated herein by reference. Examples of base-stable anchor groups are illustrated below.

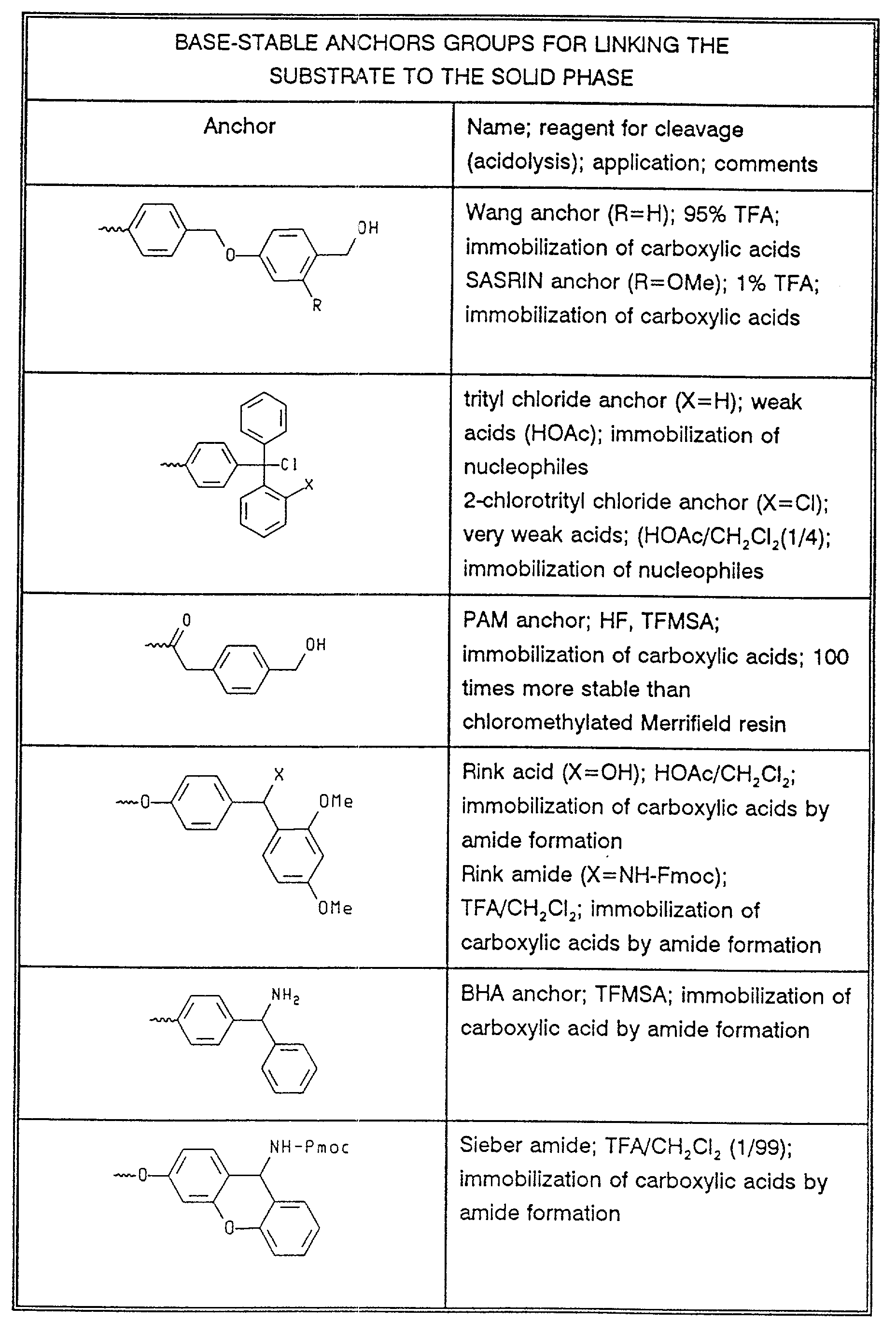

As used in this application the symbol "Ⓟ " means a support; the symbol "IN -CHO" means a compound of Formula II; "IN" means an indole bound to a support; the symbols "Ⓑ " and "© " mean organic radicals.

As used in this application the term "support" means a material which is capable of being joined to a linking means and is inert to the reaction conditions employed in the synthesis of the amide-like compounds of this invention. The support joined by linking means to an indole must be separable from the reaction mixture after reaction with an acid derivative as illustrated in scheme 1, compound 2, below. Examples of supports include, but are not limited to, resins, Grapon pins, controlled pore glass, paper disks and silicon chips. The preferred support is a resin; a preferred resin is aminomethylated polystyrene.

As used in this application the term "resin" means a solid polymer which is capable of being attached to an indole aldehyde. A preferred resin is polystyrene in the form of beads 100400 mesh in size which is aminomethylated by the procedure of Merrifield (JOC (1978), 43, 2845). Aminomethylated resins are commercially available from Calbiochem-Novabiochem Corp., San Diego, CA. The choice of resin is not critical except that it must be solid and chemically stable under the reaction conditions of the present invention.

As used in this application, "amide-like compounds" include aromatic and aliphatic amides, sulfonamides, phosphoramides, substituted ureas, thioureas, guanidines and urethanes.

Preparation of an example of the indole resin of this invention is illustrated in Example 1, below.

Optionally substituted indole-3-carboxaldehyde is reacted with a haloacetate ester in the presence of base in a suitable solvent until the reaction is completed. The ester product is hydrolyzed with dilute caustic to form 3-formyl-indole-1-yl-acetic acid.

The indole acetic acid product is reacted with aminomethyl resin suspended in an inert solvent in the presence of a dehydrating agent such as carbodiimide at room temperature for a sufficient time to complete the reaction to yield the compound of Formula I.

The utility of the compounds of the invention is illustrated in Scheme 1 and Examples 2-19 below.

In a first step the compound of Formula I is reacted with an amino compound under reducing conditions to form a resin bound secondary aminomethylene structure. A mixture of tetramethyl ammonium triacetoxyborohydride in dichloromethane, followed by sodium cyanoborohydride has been found to be effective.

The resin bound amine is then reacted with an acid halide, preferably an acid chloride in a suitable solvent such as methylene chloride with an acid acceptor such as triethylamine to yield a resin bound amide. The acid halide may be any aliphatic or aromatic acid halide or a sulfonic acid halide or thiourea or isocyanate group may also be used to prepare the resin bound amide.

In a final step, the resin bound amide is removed from the resin by reaction with a strong organic acid such as trifluoroacetic acid to yield the final amide-type product.

The above reaction steps are suitable for the preparation of multiple sequential syntheses described above to prepare small quantities of amide-type structures for pharmaceutical or other screening and evaluation procedures.

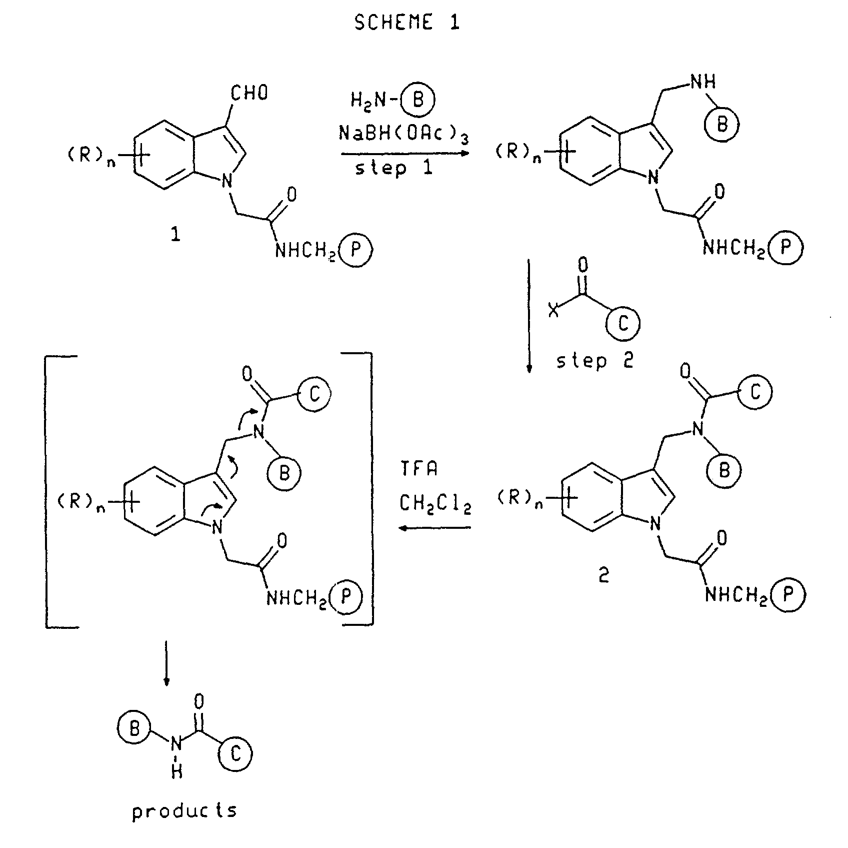

### EXAMPLE 1

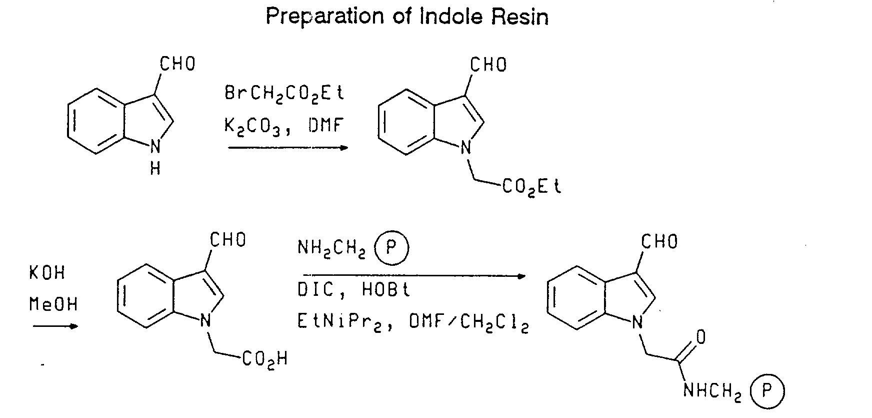

A mixture of indole-3-carboxaldehyde (1.0 g, 6.9 mmol), ethyl bromoacetate (1.4 g, 8.3 mmol) and milled potassium carbonate (1.1 g, 8.3 mmol) in DMF (10 mL) was stirred at ambient temperature for 12 h and subsequently was heated at 60 °C for 60 min. The mixture was poured into water (150 mL), and the solids were collected and dried to afford 1.23 g of crude ethyl 3-formyl-indol-1-yl-acetate. This was treated with potassium hydroxide (0.47 g, 8.4 mmol) in methanol (10 mL) at reflux for 2 h. Solvents were removed, and the residue was dissolved in water and washed with ethyl acetate. The aqueous phase was acidified with 6N HCI, the solids were collected and dried. Recrystallization from ethanol afforded 0.79 g (56%) of 3-formyl-indol-1-yl-acetic acid.

Aminomethyl resin (500 mg; 0.75 meq amine/g) was suspended in dichloromethane (10 mL) and dimethylformamide (1.5 mL). 3-Formyl-indol-1-yl-acetic acid (0.15 g, 0.75 mmol), N-hydroxybenzotriazole (0.40 g, 3.0 mmol), 1,3-diisopropylcarbodiimide (0.38 g, 3.0 mmol), and N,N-diisopropylethylamine (0.15 g, 1.1 mmol) were added, and the reaction was stirred for 3 days. The resin was collected and washed with dichloromethane, MeOH, DMF, MeOH, and dichloromethane to afford 0.58 g of indole resin **1**. IR spectral data were consistent with the desired material.

### EXAMPLE 2

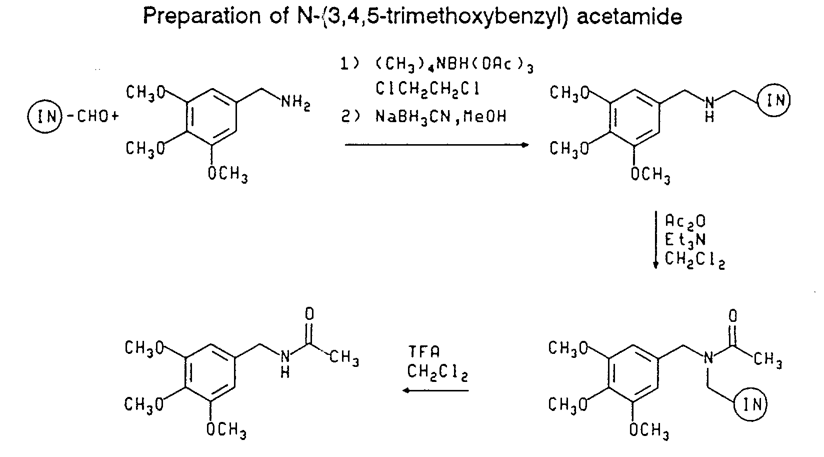

### A. Preparation of resin bound amine

To a suspension of indole resin Example 1 (4 g; 0.6 meq CHO/g) in dichloroethane (100 mL) was added 3,4,5-trimethoxybenzylamine (1.9 mL, 11 mmol) and tetramethylammonium triacetoxyborohydride (2.5 g, 11 mmol). After stirring at ambient temperature for 2 days, a mixture of sodium cyanoborohydride (3.6 g, 57 mmol) in MeOH (5 mL) was added, and the reaction was stirred for an additional 6 h. The resin was collected, washed with dichloromethane, MeOH, DMF, MeOH, and dichloromethane to afford resin bound amine. IR spectral data were consistent with the desired structure.

### B. Preparation of resin bound acetamide.

To a suspension of resin from A, above (62 mg; 0.6 meq/g) in dichloromethane (5 mL) was added triethylamine (1 mL) and acetic anhydride (0.5 mL). After stirring at ambient temperature overnight, the resin was collected and washed several times with MeOH and dichloromethane to afford resin bound acetamide **3**. IR spectral data were consistent with the desired structure.

### C. Preparation of N-(3,4,5-trimethoxybenzyl) acetamide

The resin bound acetamide from B, above was suspended in a 50% solution (v/v, 1 mL) of trifluoroacetic acid in dichloromethane, and the mixture was stirred at ambient temperature for 4 h. The supernatant solution was removed, and the resin was washed 3 times with dichloromethane. The filtrates were combined and concentrated to afford 8.0 mg (93%) of N-(3,4,5-trimethoxybenzyl) acetamide. ¹H NMR (300 MHz, CDCl₃) δ 6.50 (s, 2H), 5.82 (br, 1H), 4.35 (d, J=5.7 Hz, 2H), 3.85 (s, 6H), 3.82 (s, 3H), 2.05 (s, 3H). MS; MH⁺ 240.

### EXAMPLE 3

Using the procedure of Example 2, benzylamine was converted to N-benzylacetamide in 91% yield.

### EXAMPLE 4

Using the procedure of Example 2, d-amphetamine was converted to N-acetyl-d-amphetamine in 99% yield.

### EXAMPLE 5

Using the procedure of Example 2, 4-chlorobenzylamine was converted to N-(4-chlorobenzyl) acetamide in 94% yield.

### EXAMPLE 6

Using the procedure of Example 2, 2-(p-tolyl)ethylamine was converted to N-[2-(p-tolyl)ethyl] acetamide in 78% yield.

### EXAMPLE 7

Using the procedure of Example 2, 3,3-diphenylpropylamine was converted to N-[3,3-diphenylpropyl] acetamide in 67% yield.

### EXAMPLE 8

Using the procedure of Example 2, adamantylmethylamine was converted to the corresponding acetamide in 95% yield.

### EXAMPLE 9

Using the procedure of Example 2, 3-imidazol-1-yl-propylamine was converted to the corresponding acetamide in 97% yield.

### EXAMPLE 10

Using the procedure of Example 2, 4-(aminomethyl)pyridine was converted to the corresponding acetamide in 100% yield.

### EXAMPLE 1 1

Using the procedure of Example 2, 3-methoxypropylamine was converted to the corresponding acetamide in 97% yield.

### EXAMPLE 12

Using the procedure of Example 2, 3-isopropoxypropylamine was converted to the corresponding acetamide in 93% yield.

### EXAMPLE 13

Using the procedure of Example 2, 3-methyl-butylamine was converted to the corresponding acetamide in 94% yield.

### EXAMPLE 14

Using the procedure of Example 2, 4-(2-aminoethyl)morpholine was converted to the corresponding acetamide in 94% yield.

### EXAMPLE 14A

Using the procedure of Example 2, piperazine was converted to the TFA salt of the corresponding acetamide in 93% yield.

### EXAMPLE 14B

Using the procedure of Example 2, 2,2-diphenylethylamine was converted to the corresponding acetamide in 88% yield.

### EXAMPLE 14C

Using the procedure of Example 2, N-BOC-4-methylamino-piperidine was converted to the TFA salt of the corresponding acetamide in 86% yield.

### EXAMPLE 14D

Using the procedure of Example 2, 2-methylaminotetrahydrofuran was converted to the corresponding acetamide in 81% yield.

### EXAMPLE 14E

Using the procedure of Example 2, 1-(3-aminopropyl)-2-pyrrolidinone was converted to the corresponding acetamide in 89% yield.

### EXAMPLE 14F

Using the procedure of Example 2, 2-methoxyphenethylamine was converted to the corresponding acetamide in 77% yield.

### EXAMPLE 14G

Using the procedure of Example 2, 3,4-dichlorobenzylamine was converted to the corresponding acetamide in 80% yield.

### EXAMPLE 14H

Using the procedure of Example 2, 2-thiophenemethylamine was converted to the corresponding acetamide in 76% yield.

### EXAMPLE 15

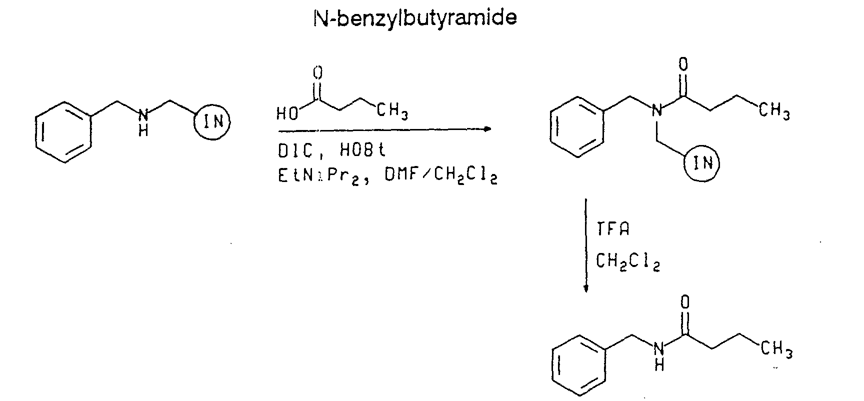

To a suspension of indole resin, Preparation 1 (0.20 g at 0.44 meq/g, Example 3) in 15% (v/v) DMF in dichloromethane (10 mL) was added butyric acid (31 µL, 0.34 mmol), N-hydroxybenzotriazole (92 mg, 0.68 mmol), N,N-diisopropylethylamine (44 µL, 0.26 mmol), and 1,3-diisopropylcarbodiimide (110 µL, 0.68 mmol). After stirring at ambient temperature for 4 days, the resin was collected, washed with dichloromethane, and dried under vacuum. The resulting resin was suspended in 50% (v/v) trifluoroacetic acid in dichloromethane (30 mL), and the mixture was stirred for 4 h. The solution was removed, and the resin was washed with dichloromethane. All filtrates were combined and concentrated, and the crude product was passed through a plug of silica gel using 50% ethylacetate/hexanes as eluent to afford 13 mg (91%) of N-benzylbutyramide.
¹H NMR (400 MHz, CDCl₃) δ 7.28 (m, 5H), 5.76 (br, 1H), 4.43 (d, J=5.6 Hz, 2H), 2.19 (t, J=7.5 Hz, 2H), 1.67 (m, 2H), 0.95 (t, J=7.5 Hz, 3H).
MS: 178 (MH⁺).

### EXAMPLE 16

### N-benzylcyclohexanecarboxamide

Using the same procedure as in the preparation of N-benzylbutyramide, cyclohexanecarboxylic acid was converted to N-benzylcyclohexanecarboxamide in 89% yield.

### EXAMPLE 17

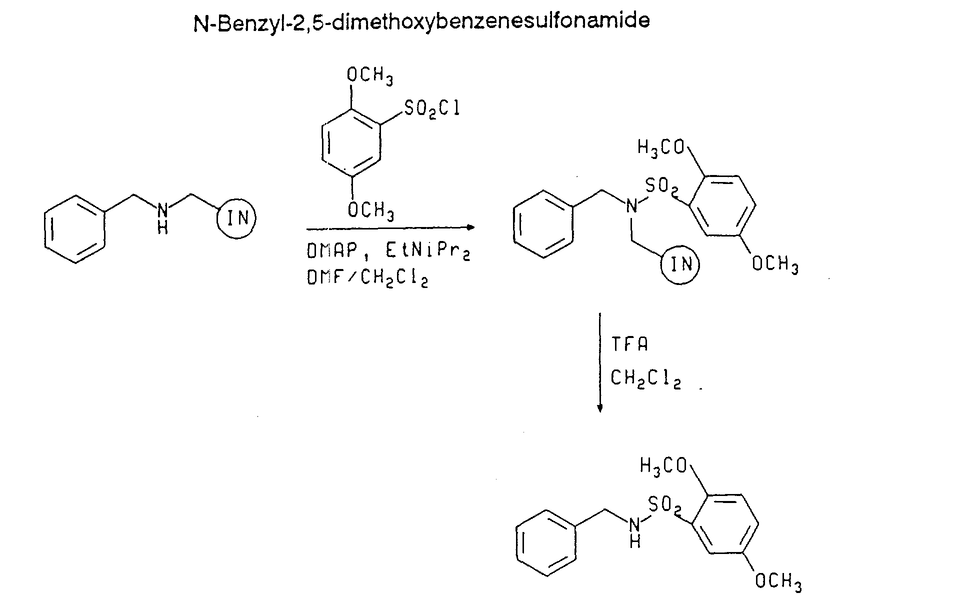

To a suspension of indole resin, Preparation 1 (115 mg at 0.44 meq/g, Example 2 in 10% (v/v) DMF in dichloromethane (10 mL) was added 2,5-dimethoxybenzenesulfonyl chloride (60 mg, 0.25 mmol), N,N-diisopropylethylamine (44 µL, 0.26 mmol), and 4-dimethylaminopyridine (2 mg), and the resulting mixture was stirred at ambient temperature for 4 days. The resin was collected, washed with dicloromethane, and treated with 50% (v/v) trifluoroacetic acid in dichloromethane (30 mL) for 4 h. The solution was removed, and the resin was washed with dichloromethane. All filtrates were combined and concentrated, and the crude product was passed through a plug of silica gel using 50% ethylacetate/hexanes as eluent to afford 14 mg (96%) of N-benzyl-2,5-dimethoxybenzenesulfonamide.
¹H NMR (400 MHz, acetone-d₆) δ 7.3-7.0 (m, 8H), 6.60 (br, 1 H), 4.08 (d, J=6.4 Hz, 2H), 3.82 (s, 3H), 3.79 (s, 3H).
MS: 308 (MH⁺).

### EXAMPLE 17A

Using the same procedure as in the preparation of N-benzyl-2,5-dimethoxybenzenesulfonamide, p-toluenesulfonyl chloride was converted to N-benzyl-4-methylbenzenesulfonamide in 99% yield.

### EXAMPLE 17B

Using the same procedure as in the preparation of N-benzyl-2,5-dimethoxybenzenesulfonamide, 4-methoxybenzenesulfonyl chloride was converted to N-benzyl-4-methoxybenzenesulfonamide in 96% yield.

### EXAMPLE 18

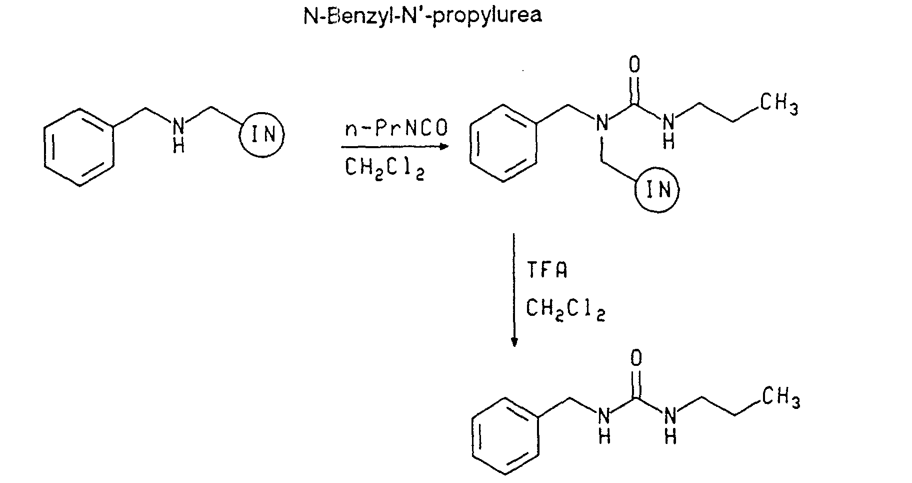

To a suspension of indole resin, Preparation 1 (180 mg at 0.44 meq/g, Example 2) in dichloromethane (10 mL) was added propyl isocyanate (37 µL, 0.40 mmol), and the resulting mixture was stirred at ambient temperature for 4 days. The resin was collected, washed with dichloromethane, and dried under vacuum. A portion of this material (170 mg) was treated with 50% (v/v) trifluoroacetic acid in dichloromethane (30 mL) for 4 h. The solution was removed, and the resin was washed with dichloromethane. All filtrates were combined and concentrated, and the crude product was passed through a plug of silica gel, eluting with chloroform then ethyl acetate, to afford 13 mg (88%) of N-benzyl-N'-propylurea.
¹H NMR (400 MHz, acetone-d₆) δ 7.3 - 7.1 (m, 5H), 5.84 (br, 1H), 5.56 (br, 1H), 4.31 (d, J=6.0 Hz, 2H), 3.08 (m, 2H), 1.40 (m, 2H), 0.80 (t, J=7.5 Hz, 3H).
MS: 193 (MH⁺).

### EXAMPLE 19

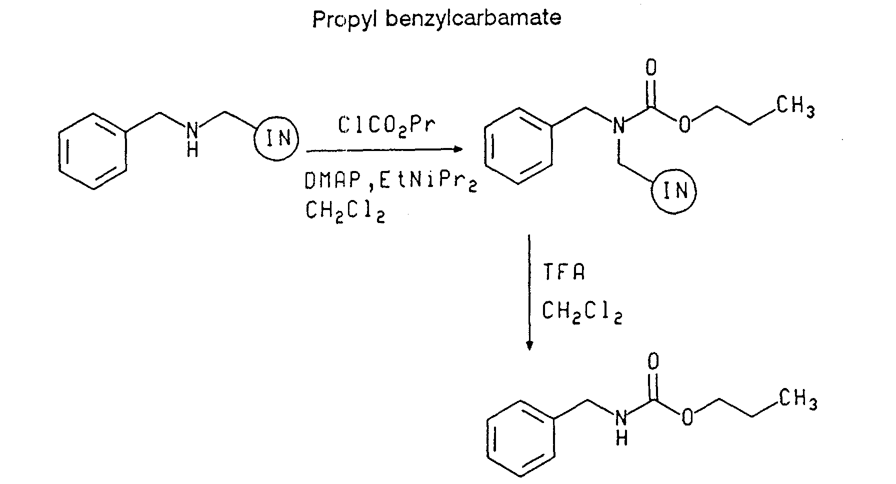

To a suspension of indole resin, Preparation 1 (200 mg at 0.44 meq/g, Example 2) in dichloromethane (10 mL) was added n-propyl chloroformate (40 µL, 0.35 mmol), N,N-diisopropylethylamine (120 µL, 0.70 mmol), and 4-dimethylaminopyridine (5 mg), and the resulting mixture was stirred at ambient temperature for 3 days. The resin was collected, washed with dichloromethane, and dried under vacuum. A portion of this material (100 mg) was treated with 50% (v/v) trifluoroacetic acid in dichloromethane (30 mL) for 4 h. The solution was removed, and the resin was washed with dichloromethane. All filtrates were combined and concentrated, and the crude product was passed through a plug of silica gel, eluting with chloroform, to afford 14 mg (80%) of propyl benzylcarbamate.
¹H NMR (400 MHz, acetone-d₆) δ 7.3 - 7.2 (m, 5H), 6.65 (br, 1H), 4.30 (d, J= 6.2 Hz, 2H), 3.96 (m, 2H), 1.58 (m, 2H), 0.89 (m, 3H).
MS: 194 (MH⁺).

### EXAMPLE 20

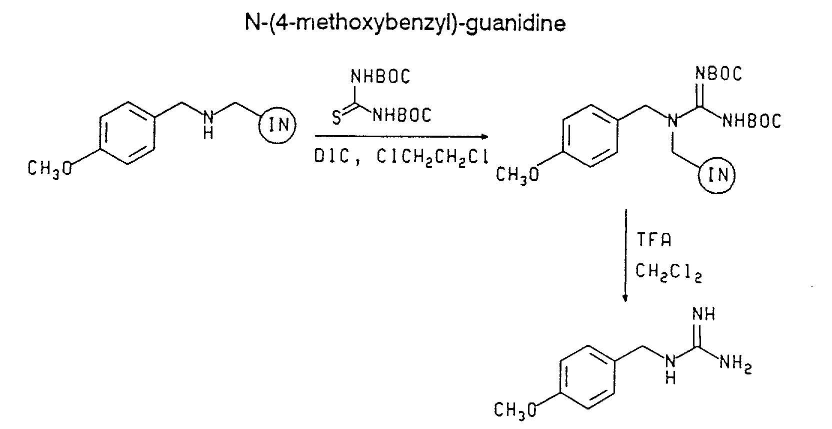

A. Using the procedure of Example 2, 4-methoxybenzylamine was converted to the resin-bound amine, which had IR spectral data consistent with the desired structure.

B. To a suspension of the resin-bound amine (0.15 g at 0.16 meq/g) in dichloroethane (10 mL) was added bis-BOC thiourea (0.070 g, 0.25 mmol) and 1,3-diisopropylcarbodiimide (40 µL, 0.68 mmol). After stirring at ambient temperature for 18 h, the resin was collected, washed with dichloromethane, and dried under vacuum. The resulting resin was suspended in 50% (v/v) trifluoroacetic acid in dichloromethane (20 mL), and the mixture was stirred for 4 h. The solution was removed, and the resin was washed with dichloromethane. All filtrates were combined and concentrated, and the crude product was passed through a plug of silica gel using ethylacetate followed by 15% methanol/acetonitrile as eluent to afford 12 mg (96%) of the bis-TFA salt of N-(4-methoxybenzyl)-guanidine.
¹H NMR (400 MHz, CD₃CN) δ 8.37 (br, 1 H), 7.3 (br, 2H), 7.24 (d, J=8.3 Hz, 2H), 6.8 (br, 1H), 6.91 (d, J=8.3 Hz, 2H), 4.25 (d, J=1.5 Hz, 2H), 3.77 (s, 3H),
MS: 180 (MH⁺).

### EXAMPLE 21

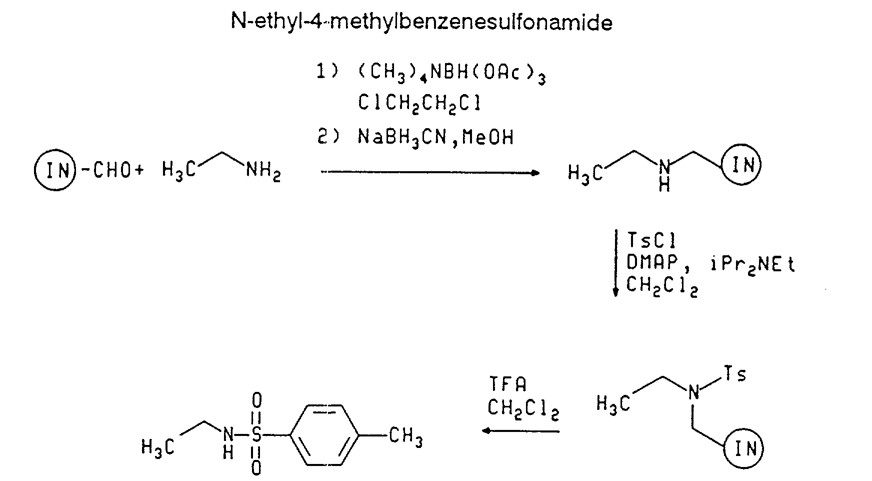

A. Using the procedure of Example 2A, N-ethylamine was converted to the resin-bound amine, which had IR spectral data consistent with the desired structure.

B. Using the procedure of Example 17A, the resin-bound N-ethylamine was converted to N-ethyl4-methylbenzenesulfonamide in 80% yield.

### EXAMPLE 22

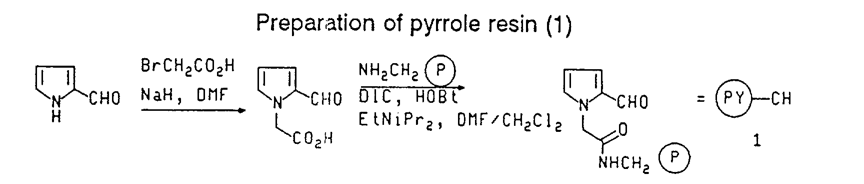

A mixture of pyrrole-2-carboxaldehyde (3.94 g, 41.4 mmol) and bromoacetic acid (8.64 g, 62.1 mmol) in DMF (100 mL) was cooled to 0°C. Sodium hydride (5.8 g of a 60% dispersion in oil, 145 mmol) was added portionwise, and the resulting heterogeneous mixture was allowed to warm to room temperature while stirring overnight. The mixture was poured into ice, and the resulting solution was extracted with dichloromethane. The aqueous phase was saturated with NaCl, extracted with ethyl acetate, dried and concentrated to a purple black solid. Chromatography on silica gel afforded 2 g (31%) of 2-formyl-pyrrole-1-acetic acid.

Aminomethyl resin (500 mg; 0.75 meq amine/g) was suspended in dichloromethane (10 mL) and dimethylformamide (1.5 mL). 2-Formyl-pyrrole-1-acetic acid (0.11 g, 0.75 mmol), N-hydroxybenzotriazole (0.40 g, 3.0 mmol), 1,3-diisopropylcarbodiimide (0.38 g, 3.0 mmol), and N,N-diisopropylethylamine (0.15 g, 1.1 mmol) were added, and the reaction was stirred for 2 days. The resin was collected and washed with dichloromethane, MeOH, DMF, MeOH, and dichloromethane to afford 0.52 g of pyrrole resin. IR spectral data were consistent with the desired material.

### EXAMPLE 23

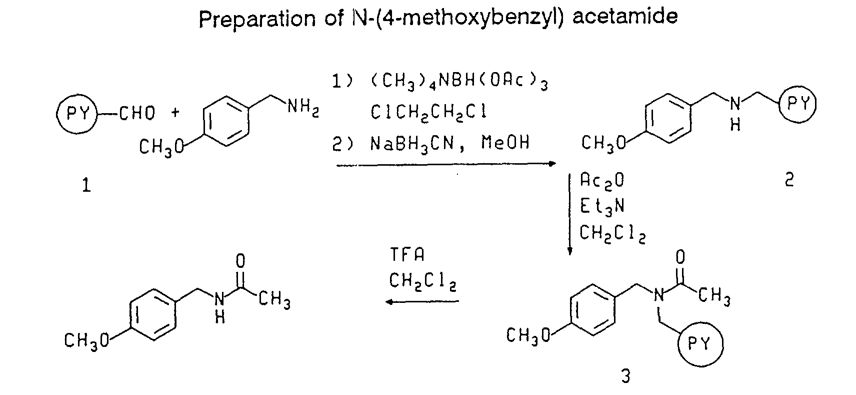

### A. Preparation of resin bound amine (2)

To a suspension of pyrrole resin from Example 22 (0.15 g; ^{∼}0.75 meq CHO/g) in dichloroethane (6 mL) was added 4-methoxybenzylamine (74 µL, 0.56 mmol) and tetramethylammonium triacetoxyborohydride (0.15 g, 0.56 mmol). After stirring at ambient temperature for 2 days, a mixture of sodium cyanoborohydride (0.18 g, 2.8 mmol) in MeOH (1 mL) was added, and the reaction was stirred for an additional 6 h. The resin was collected, washed with dichloromethane, MeOH, DMF, MeOH, and dichloromethane to afford resin bound amine. IR spectral data were consistent with the desired structure.

### B. Preparation of resin bound acetamide (3)

To a suspension of resin from procedure A (75 mg; ^{∼}0.75 meq/g) in dichloromethane (2.5 mL) was added triethylamine (0.5 mL) and acetic anhydride (0.35 mL). After stirring at ambient temperature overnight, the resin was collected and washed several times with MeOH and dichloromethane to afford resin bound acetamide. IR spectral data were consistent with the desired structure.

### C. Preparation of N-(4-methoxybenzyl) acetamide

The resin from B was suspended in a 5% solution (v/v, 3 mL) of trifluoroacetic acid in dichloromethane, and the mixture was stirred at ambient temperature ovemight. The supernatent solution was removed, and the resin was washed 3 times with dichloromethane. The filtrates were combined and concentrated to afford 8.0 mg (80%) of N-(4-methoxybenzyl) acetamide, 95% pure by HPLC.
¹H NMR and MS data support the structural assignment.

### EXAMPLE 24

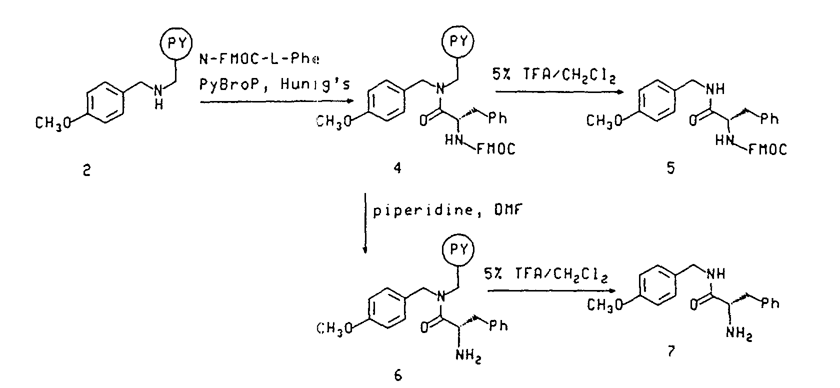

A. To a suspension of the resin bound amine **2** (116 mg; ^{∼} 0.75 meq/g) in dichloromethane (11 mL) was added sequentially N,N-diisopropylethylamine (91 mg, 120 µL), N-α-Fmoc-L-phenylalanine (136 mg, 0.35 mmol), and bromo-tris-pyrrolidinophosphonium hexafluorophosphate (160 mg, 0.35 mmol). The mixture was agitated at room temperature for 2 days, and the resin was collected and washed with warm THF to afford the resin bound amide **4**. IR spectral data were consistent with the desired structure.

B. The resin bound amide **4** (58 mg; ^{∼}0.75 meq/g) was treated with a 5% solution (v/v, 3 mL) of trifluoroacetic acid in dichloromethane for 5 h. The supernatent solution was removed, and the resin was washed 3 times with dichloromethane. The filtrates were combined and concentrated to afford 7.0 mg (33% based on the loading of aminomethyl resin) of the product **5**, for which NMR and MS data were consistent with the desired structure.

### EXAMPLE 25

The resin bound amide **4** (58 mg; ^{∼}0,75 meq/g) was treated with a 50% solution (v/v, 3 mL) of piperidine in DMF for 90 min. The resulting resin was washed with warm THF and aspirated dry to afford the deprotected amide **6**. IR spectra were consisted with the desired product. The resin bound amide **6** was then treated with a 5% solution (v/v, 3 mL) of trifluoroacetic acid in dichloromethane for 5 h. The supernatent solution was removed, and the resin was washed 3 times with dichloromethane. The filtrates were combined and concentrated to afford 6.0 mg (33% based on the loading of aminomethyl resin) of the product **7**, for which NMR and MS data were consistent with the desired structure.

### EXAMPLE 26

Using the same procedure as in the preparation of **5**, the resin bound amine **2** was coupled to N-α-Fmoc-L-alanine (110 mg, 0.35 mmol) to afford the corresponding Fmoc-benzylamide in 33% yield.

### EXAMPLE 27

Using the same procedure as in the preparation of **5**, the resin bound amine **2** was coupled to N-Fmoc-piperidine-4-carboxylic acid (120 mg, 0.35 mmol) to afford the corresponding 1-Fmoc-4-piperidinecarboxamide.

### EXAMPLE 28

Using the same procedure as in the preparation of **7**, the resin bound amine **2** was coupled to N-α-Fmoc-L-alanine (55 mg, 0.18 mmol), deprotected and cleaved to afford the corresponding Fmoc-benzylamide in 33% yield.

### EXAMPLE 29

Using the same procedure as in the preparation of **7**, the resin bound amine **2** was coupled to N-Fmoc-piperidine-4-carboxylic acid (60 mg, 0.18 mmol), deprotected and cleaved to afford the corresponding 4-piperidinecarboxamide.

### PREPARATION 1

### Indole Resin Bound Benzylamine

Indole resin from Example 1 was reacted with benzylamine by the procedure described in Example 2, Step A using benzylamine in place of 3,4,5 trimethoxybenzylamine. IR spectral data were consistent with the desired structure.

## Claims

1. A compound of the formula 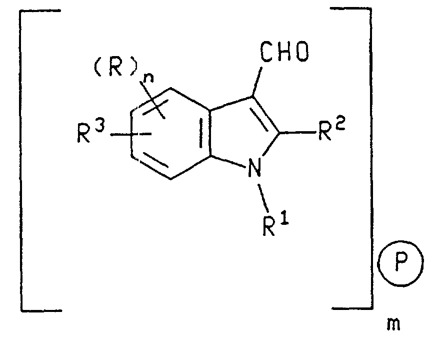 wherein
Ⓟ is a support;
one of R¹, R² and R³ is a linking means adapted to linking to Ⓟ ;
two of R¹, R² and R³ which are not a linking means are independently selected from H, C₁-C₆ alkyl and C₁-C₆ alkoxy;
each R is independently selected from F, Cl, Br, C₁-C₆ alkyl and C₁-C₆ alkoxy;
n is zero to three; and
m is a number such that 0.1 to 1.0 milliequivalent aldehyde is present for each gram of Ⓟ .

2. A compound of the formula 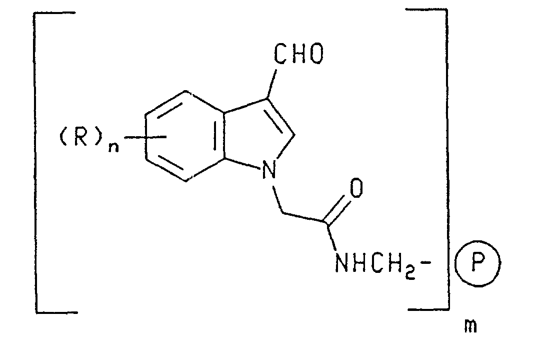 wherein
Ⓟ is a support; and
each R is independently selected from F, Cl, Br, C₁-C₆ alkyl and C₁-C₆ alkoxy;
n is zero to four; and
m is a number such that 0.1 to 1.0 milliequivalent aldehyde is present for each gram of Ⓟ .

3. A compound of claim 2 wherein n is zero.

4. A compound of claim 3 wherein approximately 0.75 milliequivalents of aldehyde are present for each gram of support.

5. A compound of claim 2 wherein said support is polystyrene, Wang Resin or Rink resin.

6. A method for the synthesis of an amide type compound which comprises:
a) reacting a primary amino compound under reducing conditions with the compound of claim 1;
b) reacting the product of step (a) with an acid chloride;
c) cleaving the product of step (b) to yield said amide type compound.

7. A compound of the formula 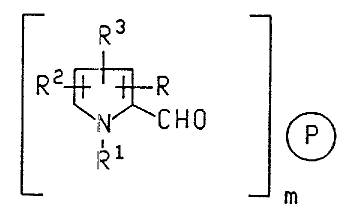 wherein:
Ⓟ is a support;
one of R¹, R² and R³ is a linking means adapted to linking to Ⓟ ;
two of R¹, R² and R³ which are not a linking means are independently selected from H, C₁-C₆ alkyl and C₁-C₆ alkoxy;
each R is independently selected from H, F, Cl, Br, C₁-C₆ alkyl and C₁-C₆ alkoxy;
m is a number such that 0.1 to 1.0 milliequivalent aldehyde is present for each gram of Ⓟ .

8. A compound of the formula 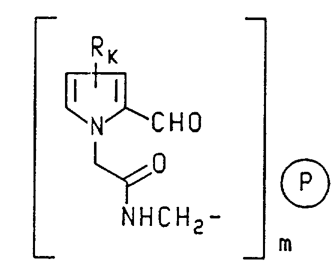 wherein Ⓟ is a support;
K is 0,1 or 2;
each R is independently selected from F, Cl, Br, C₁-C₆ alkyl and C₁-C₆ alkoxy; and
m is a number such that 0.1 to 1.0 milliequivalents aldehyde is present for each gram of Ⓟ .

9. A method for the synthesis of an amide type compound which comprises:
a) reaching a primary amino compound under reducing conditions with a compound of claim 7;
b) reacting the product of step (a) with an acid chloride;
c) cleaving the product of step (b) to yield said amide type compound.
